**Europäisches Patentamt**

**European Patent Office**

**Office européen des brevets**

(19)

(11) Veröffentlichungsnummer: **0 001 085**
**B1**

(12)

# EUROPÄISCHE PATENTSCHRIFT

(21) Anmeldenummer: **78100769.5**

(22) Anmeldetag: **28.08.78**

(51) Int. Cl.³: **C 07 C 143/665,**
**C 07 C 139/14**

(54) Verfahren zur Abtrennung von 1-Amino-4-bromanthrachinon-2-sulfonsäure

(30) Priorität: **10.09.77 DE 2740888**

(43) Veröffentlichungstag der Anmeldung:
**21.03.79 Patentblatt 79/06**

(45) Bekanntmachung des Hinweises auf die Patenterteilung:
**23.07.80 Patentblatt 80/15**

(84) Benannte Vertragsstaaten:
**BE CH DE FR GB LU NL**

(56) Entgegenhaltungen:
**FR - A - 977 423**
**Chemical Abstracts, Vol. 65, Seite 670,**
**LADISLAV HRUSKA, Czech. 115,650**
**Chemical Abstracts, Vol. 82, Seite 486, 31183 j.**

(73) Patentinhaber: **Bayer AG**
**Zentralbereich Patente, Marken und Lizenzen**
**D - 5090 Leverkusen 1, Bayerwerk (DE)**

(72) Erfinder: **Leister, Heinrich, Dr.**
**Treptower Strasse 8**
**D - 5090 Leverkusen (DE)**
**Dittmar, Helmut, Dr.**
**Zedernwerk 6**
**D - 5090 Leverkusen (DE)**
**Schönhagen, Hubert**
**Im Schwarzbroich 15**
**D - 5068 Odenthal (DE)**

Verfahren zur Abtrennung von 1-Amino-4-bromanthrachinon-2-sulfonsäure

Die Erfindung betrifft ein Verfahren zur Abtrennung von 1 - Amino - 4 - bromanthrachinon - 2 - sulfonsäure oder deren Alkalimetallsalzen aus bei der Bromierung von 1 - Amino - anthrachinon - 2 - sulfonsäure in Schwefelsäure erhaltenen Reaktionsgemischen.

1 - Amino - 4 - bromanthrachinon - 2 - sulfonsäure (im nachfolgenden Bromaminsäure genannt) ist ein wichtiges Zwischenprodukt für Anthrachinonfarbstoffe (vgl. Ullmans Encyklopädie der technischen Chemie, 4. Auflage, Band 7, S. 639—640). Zu ihrer Herstellung wird 1 - Amino - anthrachinon mit Schwefeltrioxid oder Chlorsulfonsäure in Schwefelsäure oder in Gegenwart von inerten organischen Lösungsmitteln sulfoniert (DT—PS 263 395 und 484 997, US—PS 3 428 659, japanische Offenlegungsschrift 49 076 848) und die erhaltene 1 - Amino - anthrachinon - 2 - sulfonsäure in wäßrigem oder wäßrigorganischem Medium oder in Schwefelsäure bromiert (FIAT 1 313 II, Seite 214, U.S—PS 2 413 790 und 3 428 659, japanische Offenlegungsschrift 49 076 848).

Bei der Bromierung in Schwefelsäure gemäß der japanischen Offenlegungsschrift erfolgt die Aufarbeitung des Reaktionsgemisches durch Verdünnen mit einem großen Überschuß an Wasser. Durch Zugabe von Alkalilauge fällt man die Bromaminsäure in Form ihres Alkalisalzes, aus, welches dann durch Filtration isoliert wird.

Dieses Aufarbeitung hat den Nachteil, daß Filtrate mit hoher Salzlast anfallen, die eine erhebliche Abwasserbelastung darstellen. Die Aufbereitung von Abwässern mit hoher Salzlast ist z.B. durch Eindampfen möglich. Dieser Prozeß verursacht aber nicht nur einen hohen technischen Aufwand, es entstehen auch große Mengen Rückstand an durch organische Stoffe verunreinigtem Salz. So erhält man beispielsweise bei Nacharbeitung des Beispiels 1 der japanischen Offenlegungsschrift 49 076 848 pro kg Bromaminsäure etwa 7 kg Natriumsulfat bzw. Natriumhydrogensulfat infolge Neutralisation der Schwefelsäure mit Natronlauge.

Ein weiterer Nachteil der Aufarbeitung durch Verdünnen mit einem großen Überschuß an Wasser besteht darin, daß nicht nur die Bromaminsäure ausgefällt wird, sondern auch Nebenprodukte der Sulfonierung und der Bromierung, wie z.B. andere Sulfonsäuren und 1 - Amino - 2 - und - 4 - brom - anthrachinon sowie insbesondere - Amino - 2,4 - dibromanthrachinon. Diese Verunreinigungen stören bei den meisten Umsetzungen der Bromaminsäure zu Farbstoffen, und müssen daher vorher entfernt werden.

Nach den Angaben der japanischen Offenlegungsschrift 49 076 848 besteht die Möglichkeit, die nicht sulfonierten Anteile, wie 1 - Amino - 2,4 - dibromanthrachinon, durch Ausschütteln mit o-Dichlorbenzol und Abtrennen der organischen Phase zu entfernen. Hierbei ist nicht nur die Phasentrennung ein in technischer Hinsicht problematischer Verfahrensschritt, es ist zur Reinhaltung das Abwassers auch notwendig, die wäßrige Phase durch Destillation von den immer vorhandenen Anteilen an organischen Lösungsmitteln zu befreien.

Eine weitere Möglichkeit zur Entfernung von Verunreinigungen ist die Klärfiltration (vgl. z.B. Houben-Weyl, Methoden der organischen Chemie, 4. Auflage, Band I/1, Seite 185 ff). Hierzu löst man die aus dem Reaktionsgemisch ausgefällte Bromaminsäure in heißem Wasser, fügt Aktivkohle und/oder ein Filterhilfsmittel hinzu, filtriert und fällt aus dem Filtrat mit Hilfe von Alkalimetallsalzen die Bromaminsäure wieder aus. Vielfach wird die geforderte Qualität erst durch zweifache Klärung erreicht, beispielsweise im schwach suaren oder schwach alkalischen Medium. Der Klärprozeß verursacht damit einen beträchtlichen Aufwand an Zeit, technischer Ausrüstung und Kosten. Darüber hinaus entstehen große Volumina an aufzubereitenden Abwässern.

Es wurde nun gefunden, daß man 1 - Amino - 4 - bromanthrachinon - 2 - sulfonsäure oder deren Alkalimetallsalze aus dem bei ihrer Herstellung durch Bromierung von 1 - Amino - anthrachinon - 2 - sulfonsäure in Schwefelsäure entstehenden Reaktionsgemisch in guter Reinheit und auf abwassertechnisch einfache Weise isolieren kann, wenn man durch Mischen des Reaktionsgemisches mit gegebenenfalls Schwefelsäure-haltigem Wasser eine Schwefelsäure-Konzentration von 60 bis 85 Gew.% einstellt, Wobei die 1 - Amino - 4 - bromanthrachinon - 2 - sulfonsäure als Sulfat ausfällt, dieses isoliert und durch Einwirkung von Wasser, gegebenenfalls in Gegenwart von Alkalimetallhydroxiden, - oxiden oder -salzen, in die 1 - Amino - 4 - bromanthrachinon - 2 - sulfonsäure oder deren Alkalimetallsalze überführt.

Das Reaktionsgemisch, aus dem die Bromaminsäure isoliert wird, kann auf beliebige Art durch Bromierung von 1 - Aminoanthrachinon - 2 - sulfonsäure in Schwefelsäure entstanden sein, beispielsweise durch Bromierung in wäßriger, wasserfreier oder $SO_3$-haltiger Schwefelsäure. Man kann auch das Reaktionsgemisch verwenden, welches ausgehend von 1 - Amino - anthrachinon durch Sulfonierung mit Schwefeltrioxid oder Chlorsulfonsäure und anschließende Bromierung im Eintopfverfahren erhalten wird. Schließlich kann das nach der japanischen Offenlegungsschrift 49 076 848 enstehende Reaktionsgemisch eingesetz werden. Anorganische Salze, wie Natriumsulfat, oder Katalysatoren, wie Jod, stören nicht.

Die Einstellung der Schwefelsäurekonzentration kann in beliebiger Weise vorgenommen werden, beispielsweise durch Einrühren des Reaktionsgemisches in das gegebenenfalls schwefelsäurehaltige Wasser oder umgekehrt. Hierbei erhält man eine Schwefelsäurekonzentration von 60 bis 85 Gew.-%, vorzugsweise 65 bis 80 Gew.-%.

Die Ausfällung kann mit oder ohne Außenkühlung erfolgen. Bewährt hat sich die Ausfällung bei Temperaturen im Bereich etwa 50 bis etwa 90°C, vorzugsweise 50 bis 60°C, durchzuführen. Zur Erzielung einer besseren Kristallform sind bisweilen höhere Temperaturen als 90°C vorteilhaft, beispielsweise bis etwa 120°C.

Nach dem Filtrieren kann man den Filterkuchen zur Entfernung der anhaftenden Mutterlauge mit verdünnter Schwefelsäure nachwaschen, deren Konzentration swischen etwa 60 Gew.-% und der Fällungskonzentration liegen sollte.

Aus dem isolierten Sulfat kann die Bromaminsäure durch Einwirkung von Wasser in Freiheit gesetzt werden. Dazu kann man das Filtergut mit Wasser, dem atwas Alkalimetallsalz, wie Natriumchlorid, Kaliumchlorid, und/oder Natriumsulfat, zugefügt wurde, neutral waschen. Für die praktische technische Anwendung vorteilhafter ist jedoch, den Filterkuchen mit Wasser anzurühren, gegebenenfalls unter Zusatz von Alkalimetallsalz, wie beispielsweise Natrium-, Kaliumchlorid und/oder Natriumsulfat, oder von Alkalien, wie Natron- und/oder Kalilauge und/oder Natriumcarbonat. Hierbei sollte gut gerührt und gegebenenfalls nachgeheizt werden, z.B. auf etwa 90°C. Dann wird abfiltriert, mit verdünnter Salzlösung gewaschen und getrocknet.

Die so isolierte Bromaminsäure bzw. deren Alkalimetallsalze sind von guter Qualität (etwa 93—95 %ig bezogen auf die freie Säure). Insbesondere im Falle der Herstellung der Bromaminsäure aus 1 - Aminoanthrachinon durch Sulfonierung und Bromierung im Eintopfverfahren (vgl. Beispiel 1, 3, 5 und 6) sind Ausbeute und Qualität bei Isolierung nach dem vorliegenden Verfahren weit besser als bei Einrühren des Reaktionsgemisches in viel Wasser und anschließender Klärung (vgl. Beispiel 2).

Ein besonderer Vorteil des erfindungsgemäßen Verfahrens besteht darin, daß die bei der Filtration des Bromaminsäure-Sulfats anfallende etwa 60- bis 80 Gew.-%ige Schwefelsäure nach bekannten Verfahren aufkonzentriert werden kann, (vgl. z.B. Ullmanns Encyklopädie der technischen Chemie, 3. Auflage, Band 15, Seite 422 ff).

Ein weiterer Vorteil liegt darin, daß die beim Anrühren des schwefelsauren Nutschkuchens mit Wasser erhaltene verdünnte Schwefelsäure als Mischungskomponente wieder verwendet werden kann. Durch diese Recyclisierung wird die Abwasser-belastung weiter verringert. Insgesamt werden durch Aufkonzentrierung und Recyclisierung etwa 80% der eingesetzten Schwefelsäure wiedergewonnen.

Im verfahrensgemäßen Temperatur- und Konzentrationsbereich ist das Bromaminsäure-Sulfat überraschend stabil. Selbst wenn die Temperatur bei Verdünnung des Reaktionsgemisches mit gegebenenfalls Schwefelsäurehaltigem Wasser auf 110—120°C ansteigt (vgl. Beispiel 1), wird es chemisch nicht verändert. Dies war nicht vorauszusehen, da aus den DT-PSen 263 395 und 266 563 bekannt ist, daß Bromaminsäure in 60-bzw. 78 %iger Schwefelsäure bereits bei Temperaturen von etwa 150°C die Sulfonsäuregruppe verliert, wobei zusätzlich das Bromatom von der 4- in die 2-Stellung verlagert werden kann.

In den folgenden Beispielen bedeuten die Prozente Gewichtsprozente. Die Beispiele dienen zur Erläuterung des erfindungsgemäßen Verfahrens, ohne es jedoch auf diese Beispiele einzuschränken.

Beispiel 1

In ein Gemisch von 200 ml 20 %igem Oleum und 100 g wasserfreiem Natriumsulfat trägt man unter Rühren 150 g 96 %iges 1 - Aminoanthrachinon ein und erhitzt innerhalb 1 Stunde auf 130°C. Man hält diese Temperatur 2 Stunden lang, setzt 120 ml 20 %iges Oleum und 50 g wasserfreies Natriumsulfat zu und verrührt weitere 3 Stunden bei 130°C. Die Sulfonierung ist dann praktisch beendet. Zur Bromierung kühlt man auf 80°C ab, fügt 0,2 g Jod und ca. 0,5 ml eines Entschäumers hinzu und läßt bei 80°C innerhalb von 9 Stunden 21 ml Brom portionsweise zulaufen. Der Gehalt an 1 - Aminoanthrachinon - 2 - sulfonsäure in einer aufgearbeiteten Probe liegt danach unter 1%.

Zur Aufarbeitung entfernt man zunächst überschüssiges Brom durch kurzes Evakuieren und läßt dann innerhalb 1 Stunde 140 ml Wasser zutropfen, wobei die Temperatur von 80°C auf 110°C ansteigt. Man saugt das Bromaminsäure-Sulfat bei 50—60°C ab und wäscht mit 300 ml 60 %iger Schwefelsäure nach. Mutterlauge und Wäsche werden vereinigt und ergeben 855 g einer 65 %igen Schwefelsäure, die nach bekannten Verfahren aufkonzentriert werden kann.

Den Nutschkuchen (668 g) rührt man in 1800 ml Wasser an, stellt durch Zugabe von 308 ml 50 %iger Natronlauge einen pH-Wert von 8 ein und erhitzt 1 Stunde auf 90°C. Dann kühlt man auf 60°C ab, saugt das Natriumsalz der Bromaminsäure ab, wäscht es mit 1200 ml einer 1,5 %igen Natriumsulfatlösung und trocknet es. Man erhält 234,6 g mit folgender Analyse:

87,5 % Bromaminsäure

0,5 % 1-Aminoanthrachinon-2-sulfonsäure

0,4 % unsulfonierte Bestandteile

7,1 % Wasser

Die Ausbeute an Bromaminsäure beträgt demnach 83,2 - der Theorie, bezogen auf 1 - Aminoanthrachinon.

### Beispiel 2

Vergleichsversuch: Sulfonierung und Bromierung nach den Angaben des Beispiels 1, Aufarbeitung durch Einrühren in einen großen Überschuß an Wasser und Klärung.

Man rührt das nach den Angaben des Beispiels 1 erhaltene Reaktionsgemisch in 1400 ml Wasser ein, saugt ab, rührt den Nutschkuchen in 2000 ml Wasser an und stellt durch Zugabe von ca. 100 ml 50 %iger Natronlauge auf pH 8—9. Zur Klärung heizt man auf 95—100°C, gibt eine Mischung von je 12 g Aktivkohle und Kieselgur hinzu, filtriert über eine vorgeheizte Nutsche und wäscht mit 400 ml heißem Wasser nach. Man salzt das Natriumsalz der Bromaminsäure mit.

36 g Natriumsulfat aus, saugt bei etwa 50°C ab, wäscht mit 1600 ml 1,5 %iger Natriumsulfatlösung und trocknet. Man erhält 187,2 g mit folgender Analyse:

88,2 % Bromaminsäure

0,5 % 1-Aminoanthrachinon-2-sulfonsäure

2,0 % unsulfonierte Bestandteile

4,7 % Wasser

Die Ausbeute an Bromaminsäure beträgt demnach nur 66,9 % der Theorie, bezogen auf 1 - Aminoanthrachinon.

### Beispiel 3

In das nach Beispiel 1 erhaltene Reaktionsgemisch läßt man bei 50—60°C 220 ml 60 %ige Schwefelsäure eintropfen, saugt das Bromaminsäure-Sulfat bei 50—60°C ab und wäscht mit 300 ml 60 %iger Schwefelsäure nach. Mutterlauge und Wäsche werden vereinigt und ergeben 1094 g einer 72 %igen Schwefelsäure, die nach bekannten Verfahren aufkonzentriert werden kann.

Die weitere Aufarbeitung erfolgt nach den Angaben des Beispiels 1. Man erhält 233,4 g 1 - amino - 4 - bromanthrachinon - 2 - sulfonsaures Natrium mit folgender Analyse:

85,2 % Bromaminsäure

0,5 % 1-Aminoanthrachinon-2-sulfonsäure

0,4 % unsulfonierte Bestandteile

8,2 % Wasser

Die Ausbeute an Bromaminsäure be— trägt demnach 80,5 % oder Theorie, bezogen auf 1 - Aminoanthrachinon.

### Beispiel 4

In ein Gemisch von 110 ml 96 %iger Schwefelsäure und 145 ml 20 %igem Oleum trägt man 200 g 1 - Aminoanthrachinon - 2 - sulfonsäure (Qualität 84,8 %) so ein, daß nach dem Eintragen die Temperatur 65°C beträgt. Man fügt 0,2 g Jod hinzu und bromiert, indem man bei 70—80°C während 9 Stunden 21 ml Brom portionsweise zugibt. Nach Entfernung überschüssigen Broms im Vakuum läßt man in das Reaktionsgemisch bei 50—60°C 340 g 30 %ige Schwefelsäure eintropfen, rührt 1/2 Stunde nach, saugt das Bromaminsäure-Sulfat ab und wäscht es mit 300 ml 60 %iger Schwefelsäure nach. Mutterlauge und Wäsche werden vereinigt und ergeben 810 g einer 68 %igen Schwefel-säure, die aufkonzentriert werden kann.

Den Nutschkuchen (604 g) rührt man in 300 ml Wasser an und saugt ab. Man erhält 390 g Filtrat mit einem Anteil von 32,5 % Schwefelsäure, welches zum Verdünnen eines neuen Reaktionsgemisches verwendet werden kann. Da sich diese Recyclisierung beliebig oft wiederholen läßt, ist es möglich, durch Aufkonzentrierung und Recyclisierung insgesamt ca. 80 % der eingesetzten Schwefelsäure auszuschleusen.

Der Nutschkuchen (495 g) wird nun in 2000 ml Wasser angerührt, die Suspension mit 154 ml 50 %iger Natronlauge auf pH 9 gestellt, 1/4 Stunde auf 90°C erhitzt, auf 50—60°C abgekühlt, abgesaugt, mit 1200 ml 1,5 %iger Natriumsulfatlösung gewaschen und getrocknet. Man erhält 242 g Natriumsalz der Bromaminsäure mit folgender Analyse:

85,2 % Bromaminsäure

Spur 1 - Aminoanthrachinon - 2 - sulfonsäure

0,4 % unsulfonierte Bestandteile

8,1 % Wasser

Die Ausbeute an Bromaminsäure beträgt demnach 92,4 % der Theorie, bezogen auf 1 - Aminoanthrachinon - 2 - sulfonsäure.

### Beispiel 5

In ein Gemisch aus 200 g 4 %igem Oleum, 30 g wasserfreiem Natriumsulfat und 46 g 97 %igem 1 - Aminoanthrachinon läßt man bei 130°C 44 g Chlorosulfonsäure eintropfen. Man verrührt 3 Stunden bei 130—135°C, um die Sulfonierung zu beenden, kühlt dann auf 80°C ab und verdünnt bei dieser Temperatur vorsichtig mit 235 g 92 %iger Schwefelsäure. Zur Bromierung fügt man 0,1 g Jod hinzu und läßt während 9 Stunden bei 80°C 38 g Brom portionsweise zutropfen. Zur Aufarbeitung entfernt man zunächst überschüssiges Brom mittels Vakuum und läßt dann 100 g Wasser zutropfen, um das Bromaminsäure-Sulfat auszufällen. Es wird abgesaugt und mit 60 %iger Schwefelsäure gewaschen. Den Nutschkuchen rührt man in 600 ml Wasser an, stellt mit Natronlauge auf pH 7, saugt das Natriumsalz der Bromaminsäure ab, wäscht es mit 400 g 1,5 %iger Natriumsulfatlösung und trocknet. Erhalten werden 72,8 g mit einer Qualität von 84,0 % an Bromaminsäure. Das enspricht einer Ausbeute von 80% der Theorie, bezogen auf 1 - Aminoanthrachinon.

### Beispiel 6

Das nach Beispiel 1 durch sulfonierung und Bromierung von 1 - Aminoanthrachinon erhaltene Reaktionsgemisch wird bei 60°C beginnend in ein Gemisch von 400 ml 30 %iger Schwefelsäure und 4 ml technischer Bisulfitlösung eingerührt, wobei die Temperatur auf etwa 80°C ansteigt. Man kühlt auf 40°C ab, saugt ab und wäscht mit 300 ml 60 %iger Schwefelsäure. Mutterlauge und Wäsche werden vereinigt und ergeben 1080 g einer 60 %igen Schwefelsäure, die nach bekannten Verfahren aufkionzentriert werden kann.

Die Aufarbeitung des Nutschkuchens erfolgt nach den Angaben des Beispiels 1. Man erhält 230,4 g 1 - amino - 4 - bromanthrachinon - 2 - sulfonsaures Natrium mit einer Qualität von 85,0 % an Bromaminsäure. Das entspricht einer Ausbeute von 80 % der Theorie, bezogen auf 1 - Aminoanthrachinon.

### Beispiel 7

In 150 ml 20 %iges Oleum werden 20 g wasserfreies Natriumsulfat gelöst und unterhalb 60°C 150 g 1 - Aminoanthrachinon eingetragen. Das Reaktionsgemisch wird 3 Std. auf 110°C erhitzt, auf 80°C abgekühlt und nach Zusatz von 55 ml 20 %igem Oleum weitere 3 Stunden auf 110°C erhitzt. Anschließend fügt man ca. 0,1—0,2 g Jod und bei 80°C langsam 24 ml Brom zu. Nach ca. 16 Stunden ist die Bromierung durch Erwärmen des Reaktionsgemisches unter Rückfluß auf 80°C beendet. Das überschüssige Brom wird durch Evakuieren oder Ausblasen sorgfältig entfernt und durch langsame Verdünnung mit 135 ml 30 %iger Schwefelsäure das Sulfat der 1 - Amino - 4 - bromanthrachinon - 2 - sulfonsaüre ausgefällt. Man filtriert, wäscht mit 300 ml 70 %iger Schwefelsäure und danach mit 200 ml 30 %iger Schwefelsäure. Zur Hydrolyse wird das Reaktionsprodukt in 2000 ml Wasser angerührt und mit 25 %iger Natronlauge bei 95°C auf pH=1 gestellt. Dabei tritt Lösung ein. Nach weiterem Zusatz von Natronlauge bis pH=7 kristallisiert das Natriumsalz der 1 - Amino - 4 - bromanthrachinon - 2 - sulfosäure aus. Man stellt mit wenig Soda auf pH 8—9, filtriert und wäscht mit einer verdünnet Natriumsulfatlösung. Ausbeute 236 g Natriumsalz mit einem Reingehalt von 86,7 %.

### Beispiel 8

In 150 ml 20 %iges Oleum werden unter Kühlung (Temperatur <60°C) 150 g 1 - Aminoanthrachinon eingetragen und das Reaktionsgemisch in ca. 1 Stunde auf 110°C erhitzt. Diese Temperatur wird ca. 2 Stunden lang gehalten, dann bei 90°C 55 ml 20 %iges Oleum langsam zugesetzt und die Sulfonierung bei 110°C fortgeführt. Nach Beendigung der Reaktion (Dauer mindestens 2 Stunden) wird das Reaktionsgemisch wie unter Beispiel 7 beschsrieben, bromiert und aufgearbeitet.

Ausbeute 226 g Natriumsalz mit einem Reingehalt von 86 %.

### Beispiel 9

Die Sulfonierung wird wie in Beispiel 7 beschrieben durchgeführt. Zur Bromierung setzt man dem Reaktionsgemisch 0,15 g Jod und 62,5 g Brom zu und erhitzt in einem geschlossenen Reaktionsgefäß unter Rühren 16 Stunden lang auf 80°C. Dabei steigt der Druck auf 3,5—4 bar. Nach Abkühlung auf 60°C wird das Reaktionsgefäß belüftet, um danach überschüssiges Brom und Bromwasserstoff durch Ausblasen bzw. Evakuieren sorgfältig aus dem Reaktionsgemisch zu entfernen.

Zur Aufarbeitung werden langsam unter Rühren 108 ml 70 %ige Schwefelsäure und danach 140 ml 30 %ige Schwefelsäure zugefügt, wobei die 1 - Amino - 4 - bromanthrachinon-2-sulfonsäure in Form ihres Sulfates ausfällt.- Man filtriert, wäscht mit 375 ml 70 %iger Schwefelsäure und mit 243 ml 30 %iger Schwefelsäure.

Die weiters Aufarbeitung durch Hydrolyse erfolgt wie unter Beispiel 7 beschrieben, wobei man wie dort die 1 - Amino - 4 - bromanthrachinon - 2 - sulfonsäure in gleich guter Qualität und Ausbeute erhählt.

### Patentansprüche

1. Verfahren zur Abtrennung von 1 - Amino - 4 - bromanthrachinon - 2 - sulfonsäure oder deren Alkalimetallsalzen aus dem bei ihrer Herstellung durch Bromierung von 1 - Amino - anthrachinon - 2 - sulfonsäure in Schwefelsäure entstehenden Reaktionsgemisch, dadurch gekennzeichnet, daß man durch Mischen des Reaktionsgemisches mit gegebenenfalls Schwefelsäure-haltigem Wasser eine Schwefelsäure-Konzentration von 60 bis 85 Gew.-% einstellt, wobei die 1 - Amino - 4 - bromanthrachinon - 2 - sulfonsäure als Sulfat ausfällt, dieses isoliert und durch Einwirkung von Wasser, gegebenenfalls in Gegenwart von Alkalimetallhydroxiden, -oxiden oder -salzen, in 1 - Amino - 4 - bromanthrachinon - 2 - sulfonsäure oder deren Alkalimetallsalze überführt.

2. Verfahren nach Anspruch 1, dadurch gekennzeichnet daß man die Ausfällung der 1 - Amino - 4 - bromanthrachinon - 2 - sulfonsäure als Sulfat bei einer Temperatur im Bereich von 50 bis 90°C durchführt.

## Revendications

1. Procédé de séparation de l'acide 1 - amino - 4 - bromanthraquinone - 2 - sulfonique ou de ses sels de métaux alcalins du mélange réactionnel produit lors de leur préparation par bromation d'acide 1 - amino - anthraquinone - 2 - sulfonique dans l'acide sulfurique, caractérisé en ce qu'il consiste à ajuster la concentration en acide sulfurique à une valeur de 60 à 85 % en poids par adjonction au mélange réactionnel d'eau contenant éventuellement de l'acide sulfurique, de manière à précipiter l'acide 1 - amino - 4 - bromanthraquinone - 2 - sulfonique à l'état de sulfate, à isoler ce dernier et à le transformer par l'action de l'eau, éventuellement en présence d'hydroxydes, d'oxydes ou de sels de métaux alcalins, en acide 1 - amino - 4 - bromanthraquinone - 2 - sulfonique ou en ses sels de métaux alcalins.

2. Procédé suivant la revendication 1, caractérisé en ce que la précipitation de l'acide 1 - amino - bromanthraquinone - 2 - sulfonique à l'état de sulfate est effectuée à une température choisie dans la plage de 50 à 90°C.

## Claims

1. Process for separating off 1 - amino - 4 - bromoanthraquinone - 2 - sulphonic acid, or the alkali metal salts thereof, from the reaction mixture formed during their preparation by bromination of 1 - amino - anthraquinone - 2 - sulphonic acid in sulphuric acid, characterised in that a sulphuric acid concentration of 60% to 85% by weight is adjusted by mixing the reaction mixture with water, which optionally contains sulphuric acid, the 1 - amino - 4 - bromoanthraquinone - 2 - sulphonic acid being precipitated as the sulphate, which is isolated and converted into 1 - amino - 4 - bromoanthraquinone - 2 - sulphonic acid, or the alkali metal salts thereof, by the action of water, if appropriate in the presence of alkali metal hydroxides, alkali metal oxides or alkali metal salts.

2. Process according to Claim 1, characterised in that the precipitation of 1 - amino - 4 - bromoanthraquinone - 2 - sulphonic acid as the sulphate is carried out at a temperature in the range of 50° to 90°C.